(19) Europäisches Patentamt
European Patent Office
Office européen des brevets

(11) Veröffentlichungsnummer: **0 305 648**
**B1**

(12) **EUROPÄISCHE PATENTSCHRIFT**

(45) Veröffentlichungstag der Patentschrift: **28.03.90**

(51) Int. Cl.⁴: **C07C 309/40**

(21) Anmeldenummer: **88107523.8**

(22) Anmeldetag: **10.05.88**

# (54) Verfahren zur Herstellung von 4,4'-Dinitrostilben-2,2'-disulfonsäure.

(30) Priorität: **12.08.87 CH 3098/87**
**29.10.87 CH 4228/87**
**25.03.88 CH 1142/88**

(43) Veröffentlichungstag der Anmeldung:
**08.03.89 Patentblatt 89/10**

(45) Bekanntmachung des Hinweises auf die Patenterteilung:
**28.03.90 Patentblatt 90/13**

(84) Benannte Vertragsstaaten:
**AT BE CH DE ES FR GB IT LI NL**

(56) Entgegenhaltungen:
**EP-A- 0 026 154**

(73) Patentinhaber: **CIBA-GEIGY AG, Klybeckstrasse 141, CH-4002 Basel(CH)**

(72) Erfinder: **Guglielmetti, Leonardo, Dr., Wartenbergstrasse 6, CH-4103 Bottmingen(CH)**

(74) Vertreter: **Dipl.-Ing. Schwabe, Dr. Dr. Sandmair, Dr. Marx, Stuntzstrasse 16, D-8000 München 80(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 305 648 B1

**Beschreibung**

Die Anmeldung betrifft ein neues Verfahren zur Herstellung von 4,4'-Dinitrostilben-2,2'-disulfonsäure.

Verfahren zur industriellen Herstellung der 4,4'-Dinitrostilben-2,2'-disulfonsäure (DNS) und deren Salze sind allgemein bekannt und bestehen in der oxydativen Kondensation von 2 Mol 4-Nitrotoluol-2-sulfonsäure (p-NTSA) unter wässrigen alkalischen Bedingungen nach den alten, am Ende des letzten Jahrhunderts entwickelten Methoden. Als Oxydationsmittel werden Sauerstoff (Luft) in Gegenwart eines Katalysators oder Natriumhypochlorit beschrieben (vgl. z.B. A.G. Green und A.R. Wahl, Chemische Berichte 30, 3097-3101 (1897); 31 1079 (1898); DRP 106.961; Chemisches Zentralblatt 1900 I, 1085; DRP 113.514 und Chemisches Zentralblatt 1900 II, 703). Diese Verfahren liefern jedoch die 4,4'-Dinitrostilben-2,2'-disulfonsäure und deren Salze, trotz modernen technischen Verbesserungen, nur in verhältnismässig schlechten Ausbeuten, die zwischen 60 und 75 % liegen (vgl. z.B. DE-OS-22 58 530).

In den letzten 25 Jahren wurden daher zahlreiche Anstrengungen unternommen, um die Ausbeute dieser Kondensation unter Anwendung physikochemischer, mathematischer und analytischer Methoden sowie von Computer-Modellen zu verbessern. Diese Anstrengungen führten aber zu keinem entscheidenden Erfolg [vgl. z.B. C.A. 83, 113.377 h (1975); C.A. 85, 192. 288 z, 192.289a, 192.290 n (1976); C.A. 86, 16029c (1977); Chimie Analytique 50, 251-254 (1968) und Chimie et Industrie, Genie Chimique 101, 1439-1447 (1969)].

Besonders zu erwähnen sind die in den letzten 10 Jahren unternommenen Anstrengungen, die praktisch alle zum Ziel hatten, die grossen oekologischen Probleme, z.B. die biologisch nicht abbaubaren Mutterlaugen, durch Optimierung der Ausbeute zu lösen.

So wird gemäss der DDR-Patentschrift 240200 die wässrige Luftoxydation von 4-Nitrotoluol-2-sulfonsäure zweistufig bei zwei verschiedenen Temperaturen und zwei verschiedenen Alkalikonzentrationen durchgeführt, wobei in der ersten Stufe die 4,4'-Dinitrodibenzyl-2,2'-disulfonsäure zum Teil ausfällt. Die Ausbeuten an 4,4'-Dinitrostilben-2,2'-disulfonsäure werden mit 82 bis 85 % der Theorie angegeben, jedoch ohne Angaben über die Qualität der erhaltenen Ware.

Aus der DE-OS-3409171 ist ein Verfahren bekannt, das die wässrige Luftoxydation von 4-Nitrotoluol-2-sulfonsäure in Gegenwart von Lithium- und Hydroxylionen gegebenenfalls unter Zusatz eines Katalysators durchführt. Die Ausbeuten an 4,4'-Dinitrostilben-2,2'-disulfonsäure werden mit etwa 80 bis 90 % der Theorie angegeben. Nachteilig bei diesem Verfahren ist der zusätzliche Schritt der Abtrennung des Lithiums als Lithiumcarbonat vor der Isolierung der 4,4'-Dinitrostilben-2,2'-disulfonsäure, wobei die Wiedergewinnung des Lithiumcarbonats nur 75 bis 83 % beträgt. Ausserdem muss das wiedergewonnene Lithiumcarbonat erst in Lithiumhydroxid umgewandelt werden, bevor es wieder in dem Prozess verwendet werden kann.

In der deutschen Offenlegungsschrift 3519552 ist ein Verfahren zur Herstellung von 4,4'-Dinitrostilben-2,2'-disulfonsäuresalzen durch wässrige Luftoxydation von 4-Nitrotoluol-2-sulfonsäure beschrieben, das dadurch gekennzeichnet ist, dass man während der Reaktion Kalium-, Calcium-, und/oder Magnesiumionen in dem Masse zugibt wie 4,4'-Dinitrostilben-2,2'-disulfonsäure gebildet wird, wobei zu jedem Zeitpunkt der Reaktion die Menge der zugegebenen Kalium-, Calcium- und/oder Magnesiumionen 10 bis 150 Mol-%, bezogen auf die jeweils im Reaktionsgemisch vorhandene Menge an 4,4'-Dinitrostilben-2,2'-disulfonsäure beträgt, und das ausgefallene Salz der 4,4'-Dinitrostilben-2,2'-disulfonsäure abtrennt. Nachteilig bei diesem Verfahren ist die Aufarbeitung und Entsorgung der grossen Mengen an Base und der in grossen Mengen zugesetzten Kalium-, Calcium- und Magnesiumsalze.

In der europäischen Offenlegungsschrift 26154 ist ein Verfahren zur Herstellung von 4,4'-Dinitrostilben-2,2'-disulfonsäure und deren Salzen durch Luftoxydation von 4-Nitrotoluol-2-sulfonsäure in organischen Lösungsmitteln beschrieben. Nach diesem Verfahren werden je nach Arbeitsweise Ausbeuten von bis zu 96 % der Theorie erzielt. Nachteilig bei diesem Verfahren ist das Arbeiten in aprotischen dipolaren Lösungsmitteln deren Wiedergewinnung aufwendig ist und bekanntlich nicht ohne Verluste durchgeführt werden kann.

Es wurde nun gefunden, dass man 4,4'-Dinitrostilben-2,2'-disulfonsäure (DNS) und deren Salze in hohen Ausbeuten und unter Vermeidung der oben erwähnten Nachteile überraschenderweise durch Oxydation von 4-Nitrotoluol-2-sulfonsäure mit einem Oxidationsmittel herstellen kann, wenn man die Oxydation in flüssigem, wasserfreiem Ammoniak, dessen Alkylderivat gegebenfalls in Gegenwart von Wasser und/oder deren Mischungen untereinander durchführt.

Gegenstand der Anmeldung ist somit ein Verfahren zur Herstellung von 4,4'-Dinitrostilben-2,2'-disulfonsäure und deren Salze n der Formel

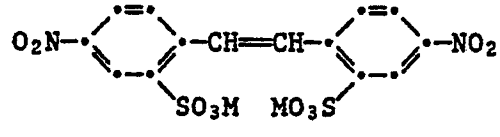

worin M Wasserstoff, ein Alkalimetallkation oder ein Ammoniumkation bedeutet, durch Oxidation von 4-Nitrotoluol-2-sulfonsäure oder deren Salze mit einem Oxidationsmittel, dadurch gekennzeichnet, dass man die Oxydation in flüssigem, wasserfreiem Ammoniak, dessen Alkylderivat gegebenenfalls in Gegenwart von Wasser und/oder deren Mischungen untereinander und in Gegenwart von starken Basen und gegebenenfalls von Katalysatoren durchführt.

Die als Ausgangsmaterial verwendete 4-Nitrotoluol-2-sulfonsäure (p-NTSA) oder deren Alkali- und Ammoniumsalze ist eine in der chemischen Industrie bekannte Verbindung, die sehr leicht durch Sulfonierung von 4-Nitrotoluol hergestellt wird. Sie kann entweder als freie Säure oder als eines ihren bekannten Salze eingesetzt werden. Diese Salze können entweder in trockener Form oder vorzugsweise auch als feuchter Presskuchen mit einem Wassergehalt von 1 bis 50 % vorzugsweise 1 bis 25 % oder einem sonstigen Vorprodukt wie der Syntheselösung oder -suspension, der aufkonzentrierten wässrigen Zubereitung, als wasserhaltiges Oel oder auch als trockenes Pulver eingesetzt werden.

Unter flüssigem, wasserfreiem Ammoniak, dessen Alkylderivat gegebenenfalls in Gegenwart von Wasser und/oder deren Mischungen untereinander die als Reaktionslösungsmittel im vorliegenden erfindungsgemässen Verfahren verwendet werden, werden vor allem folgende Lösungsmittel bzw. Kombinationen verstanden:

a) wasserfreies, flüssiges Ammoniak
b) wasserfreies, flüssiges Alkylderivat des Ammoniaks
c) Mischung aus a) und b)
d) Ammoniak und Wasser,
e) Alkylderivat des Ammoniaks und Wasser,
f) Ammoniak und ein Alkylderivat des Ammoniaks und Wasser.

Bevorzugt ist die Kombination d).

Sowohl die als Ausgangsmaterial verwendete 4-Nitrotoluol-2-sulfonsäure, als auch die als Zwischenstufe auftretende 4,4'-Dinitrodibenzyl-2,2'-disulfonsäure und das Reaktionsprodukt 4,4'-Dinitrostilben-2,2'-disulfonsäure sind z.B. unter den in diesem Verfahren genannten Reaktionsmedien, in den oben erwähnten Lösungsmitteln bzw. Kombinationen a) bis f) und besonders in der Kombination d) besser löslich als in Wasser oder aprotischen dipolaren Lösungsmitteln. Dadurch kann die, heute in der Industrie in grosser Verdünnung ablaufende Reaktion viel konzentrierter z.B. schon mit einem Teil einer der genannten Lösungsmittel bzw. Kombination a) bis f) pro Teil 4-Nitrotoluol-2-sulfonsäure, vorzugsweise mit 1-10 Teilen und insbesondere 3-6 Teilen einer der genannten Lösungs mittel bzw. Kombination a) bis f) durchgeführt werden; ein Umstand der aus technischer und wirtschaftlicher Sicht von grossem Vorteil ist. Desweitern ist Ammoniak billig und grosstechnisch leicht zugänglich. Dank seines tiefen Siedepunktes (-33,35°C bei 760 Torr) und seiner hervorragenden Stabilität unter den in diesem Verfahren genannten Reaktionsbedingungen, lässt es sich praktisch quantitativ zurückgewinnen und kann somit wieder in das Verfahren zurückgeführt werden.

Als Alkylderivat des Ammoniaks kommen sowohl primäre als auch sekundäre und tertiäre Amine der folgenden Formeln in Betracht:

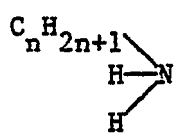
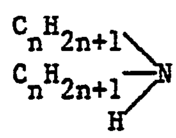
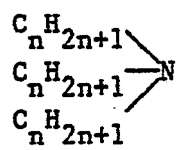

In diesen Formeln bedeutet n vor allem die Zahlen 1 bis 6 wobei es sich sowohl um einheitliche Amine (z.B. Dimethylamin) als auch um gemischte Amine (z.B. Aethyldimethylamin) handelt. Besonders bevorzugt sind Dimethylamin, Trimethylamin, Aethylamin, Diäthylamin, Triäthylamin und insbesondere Methylamin.

Die als Reaktionslösungsmittel im erfindungsgemässen Verfahren verwendeten Kombinationen d) bis f) enthalten vorzugsweise 50 bis 99 % und insbesondere 60 bis 80 % Ammoniak und/oder ein Alkylderivat des Ammoniaks bezogen auf die gesamte menge der entsprechenden Kombination. Das Verhältnis von Wasser zu Ammoniak kann dabei auf verschiedene Weise eingestellt werden, z.B. indem man Wasser, eine wässrige Ammoniaklösung mit z.B. 1 bis 30 % Ammoniakgehalt, wässrige 4-Nitrotoluol-2-sulfonsäure oder deren Salze oder flüssiges Ammoniak vorlegt und anschliessend die fehlenden Teile Ammoniak, Wasser, in Wasser gelöste Base, Ammoniakwasser bzw. Alkylderivate des Ammoniaks zugibt.

Als starke Basen kommen besonders die Alkali- oder Erdalkalimetalle, wie Lithium, Natrium, Kalium, Magnesium und Calcium sowie ihre stark basischen Verbindungen in Betracht, z.B. Hydroxide, Amide, Alkoholate, sowie stark basische Ionenaustauscher.

Als Alkoholate kommen im wesentlichen solche zur Anwendung, die sich von offenkettigen, verzweigten oder cyclischen niederen aliphatischen Alkoholen mit 1 bis 8 Kohlenstoffatomen, vorzugsweise 1 bis 4 Kohlenstoffatomen wie Methanol, Ethanol, Propanol, Butanol, Isopropanol und tert-Butanol ableiten. Diese Alkoholate werden vorzugsweise in Form der entsprechenden alkoholischen Lösung eingesetzt.

Bevorzugt werden Natrium- oder Kaliumverbindungen verwendet, wobei deren Hydroxide und Alkoholate von besonderer praktischer Bedeutung sind.

Je nach Art und Menge der verwendeten Base ist es von Vorteil die Base in einem protischen Lösungsmittel gelöst einzusetzen. Als protische Lösungsmittel kommen bevorzugt Wasser oder offenkettige, verzweigte oder cyclische niedermolekulare aliphatische Alkohole mit 1 bis 8 Kohlenstoffatomen zur Anwendung. Von besonderer Bedeutung ist die Verwendung von Methanol und/oder Wasser.

Die Base kann der Durchführung der Oxidation entweder im Reaktionskessel vorgelegt werden, wobei die p-NTSA zudosiert wird oder gleichzeitig mit p-NTSA aber über getrennte Dosiereinheiten dosiert werden, oder allein zu der vorgelegten p-NTSA zudosiert werden.

Die Menge der einzusetzenden Base kann innerhalb weiter Grenzen variieren. Bei Anwesenheit eines Katalysators können katalytische Mengen eingesetzt werden da sich die Base im Verlauf der Reaktion regeneriert. Vorzugsweise verwendet man bezogen auf p-NTSA jedoch zwischen katalytisch und äquivalent liegende Mengen an Base insbesondere 0,25 bis 0,5 Mol. Wird die Oxydation ohne Katalysator durchgeführt verwendet man bezogen auf p-NTSA mindestens äquivalente Mengen an Base. Die optimal zuzusetzende Basenmenge kann jedoch leicht durch Vorversuche festgestellt werden.

Als Katalysatoren kommen Salze, Oxide oder Hydroxide von Schwermetallverbindungen und/oder schwermetallorganischen Verbindungen wie z.B. solche des Co, Mn, Cr, Ce, Fe, Ni, Cu, Ru, Pd, Pt oder Ir in Betracht (vgl. z.B. Homogenous Catalysis by Metal Complexes, Vol. I, Chapter 2: Activation of molecular oxygen, page 79, Academic Press New York and London 1974). Von besonderer Bedeutung als Katalysatoren sind jedoch die Salze, Oxide oder Hydroxide des Mangans und/oder die manganorganischen Verbindungen wie z.B. Mangansulfat und/oder Manganacetat.

Anorganische oder organische Brom- und/oder Jod-Verbindungen wie z.B. NaJ, KJ, KBr und Ammoniumbromid können auch mit Vorteil verwendet werden.

Ferner können zusätzlich noch Phasentransferkatalysatoren oder Kronenäther eingesetzt werden, besonders in solchen Fällen, in welohen die zu verwendenden starken Basen eine ungenügende Löslichkeit in flüssigem Ammoniak aufweisen.

Beispiele von Phasentransferkatalysatoren sind: Ammoniumchlorid, Ammoniumbromid, Methylaminhydrochlorid, Cyclohexylaminhydrochlorid, Anilinhydrochlorid, Dimethylaminhydrochlorid, Di-isobutylaminohydrochlorid, Triäthylaminhydrochlorid, Triäthylaminhydrobromid, Tri-n-octylaminhydrochlorid, Benzyl-dimethylaminhydrochlorid, Tetramethyl-, Tetraäthyl-, Tetra-n-propyl-, Tetra-n-butylammoniumchlorid, -bromid und -jodid, Trimethyl-hexadecylammoniumchlorid, Benzyldimethyl-hexadecylammoniumchlorid, Benzyldimethyltetradecylammoniumchlorid, Benzyl-trimethyl-, -triäthyl- und tri-n-butylammoniumchlorid, n-Butyl-tri-n-propylammoniumbromid, Octadecyltrimethylammoniumbromid, Phenyltrimethylammoniumbromid oder -chlorid, Hexadecylpyridiniumbromid und -chlorid.

Beispiele von Kronenäther sind: 15-Crown-5; 18-Crown-6; Dibenzo-18-crown-6; Dicyclohexyl-18-crown-6; 5,6,14,15-Dibenzo-7,13-diaza-1,4-dioxacyclopentadeca-5,14-dien.

Die Menge der eingesetzten Katalysatoren kann innerhalb breiter Grenzen variieren. In manchen Fällen genügt es, wenn die Katalysatoren in Spuren vorliegen. Im allgemeinen werden jedoch die Katalysatoren bevorzugt in einer Menge von etwa 0,1 bis 15 Gewichtsprozent, bezogen auf die 4-Nitrotoluol-2-sulfonsäure bzw. deren Salze, eingesetzt.

Die Reaktionstemperatur ist im allgemeinen nicht kritisch und kann zwischen -33°C und +50°C liegen, vorzugsweise liegt sie jedoch zwischen -15°C und 30°C, insbesondere zwischen 0°C und 25°C. Wird die Reaktion bei -33°C durchgeführt, dann findet sie unter atmosphärischem Druck statt. Bei höheren Temperaturen muss die Reaktion bei dem in der Literatur (Encyclopedia of Chemical Technology, Third Edition, Volume 2, Page 474; Ullmanns Encyklopädie der technischen Chemie, 1953, Band 3, Seite 524) bekannten Dampfdruck des flüssigen Ammoniaks, des Alkylderivats des Ammoniaks bzw. des jeweiligen Gemisches von Ammoniak und/oder Alkylderivat des Ammoniaks mit Wasser durchgeführt werden.

Als Oxydationsmittel kommen reiner Sauerstoff oder seine Gemische mit inerten Gasen wie z.B. Stickstoff, und Luft in Frage. Die Oxidationsmittel werden dabei im Ueberschuss bezogen auf p-NTSA eingesetzt. Im allgemeinen verwendet man einen Ueberschuss von etwa 300 %, bevorzugt 50 % bis 100 % bezogen auf p-NTSA. Von besonderer praktischer Bedeutung ist die Verwendung von reinem Sauerstoff im geschlossenen Kreis, wobei der verbrauchte Sauerstoff dauernd ersetzt wird.

In einer bevorzugten Ausführungsform wird 1 Teil 4-Nitrotoluol-2-sulfonsäure bzw. deren Salze in Form des feuchten Presskuchens mit einer katalytischen Menge Mangan-II-Salz wie Mangansulfat und/oder Manganacetat in 0,5 bis 3 Teilen wässriger Ammoniaklösung oder Wasser suspendiert, mit 1 bis 5 Teilen flüssigem Ammoniak versetzt, so dass der Ammoniakgehalt der gesammten Reaktionsmischung 60 % bis 80 % beträgt und in Gegenwart von Sauerstoff als Oxidationsmittel und Natriumhydroxid als Base, bei Temperaturen von 0-25°C und unter Druck, umgesetzt. Die Aufarbeitung erfolgt in bekannter Weise.

Das erfindungsgemässe Verfahren liefert die 4,4′-Dinitrostilben-2,2′-disulfonsäure bzw. deren Salze in nahezu quantitativer Ausbeute und hoher Reinheit, ohne dass gefärbte Nebenprodukte entstehen.

Ein weiterer Vorteil des Verfahrens ist darin zu sehen, dass die erfindungsgemäss hergestellte DNS direkt ohne zusätzliche Reinigung und bei Verwendung von flüssigem Ammoniak auch ohne Aufarbeitung weiter zur (4-amino-4′-nitro)-stilben-2,2′-disulfonsäure und 4,4′-Diaminostilben-2,2′-disulfonsäure, einem für die Herstellung von Farbstoffen und optischen Aufhellern wichtigen Zwischenprodukt, re-

duziert werden kann. Diese Reduktion erfolgt in an sich bekannter Weise mit Wasserstoff in Gegenwart von Katalysatoren.

Die folgenden Beispiele erläutern die Erfindung ohne sie darauf zu beschränken. Teile und Prozente bedeuten Gewichtsteile bzw. Gewichtsprozente.

Beispiel 1:

In einem 1 l BUECHI-Glasautoklav ausgerüstet mit Kühl/Heiz-Mantel, Manometer, Begasungsrührer, Thermometer, Tropftrichter, Eintauchbegasungsrohr, zwei Einwegventile und zwei Rotameter für Ein- und Ausleiten von Sauerstoff und Berstscheibe (10 bar) werden bei atmosphärischem Druck und bei -40°C, 97,0 g 4-Nitrotoluol-2-sulfonsäure Natriumsalz (Aktivgehalt: 98,6 %), 4,5 g Mangan (II)-acetat Tetrahydrat und 360 g flüssiges Ammoniak vorgelegt - dies entspricht einem Verhältnis von 1 Teil p-NTSA-Na-Salz zu 3,8 Teilen Ammoniak. Der Autoklav wird geschlossen und die Innentemperatur von -33,3°C auf +5°C erhöht wobei der Innendruck auf 4 bar steigt.

In die erhaltene klare Lösung wird ein Sauerstoffstrom von 10 l/h, bei einer Rührgeschwindigkeit von 600 bis 700 UpM eingeleitet. Zusätzlich werden 21,6 g einer methanolischen 30 %igen Natriummethylat-Lösung innerhalb von 20 Minuten bei einer Innentemperatur von 5 bis 7°C zugegeben. Das erhaltene Reaktionsgemisch wird nun eine Stunde und 40 Minuten bei 5°C unter Einleiten von Sauerstoff (10 l/h) weitergerührt.

Zur Aufarbeitung wird der Innendruck im Autoklav durch partielle Verdampfung des Ammoniaks von 4 bar auf atmosphärischem Druck gesenkt, dabei sinkt die Innentemperatur von +5°C auf -27°C. Anschliessend versetzt man das Reaktionsgemisch bei atmosphärischem Druck mit 6,5 g Ammoniumchlorid und verdünnt langsam mit 400 ml Methanol, dabei fällt das Reaktionsprodukt kristallin aus. Der Sauerstoffstrom wird abgestellt und die erhaltene Suspension wird durch langsames Aufheizen auf +30°C von Ammoniak befreit. Der Rührer wird abgestellt und der Autoklav wird ausgeladen.

Das Reaktionsgemisch wird nun unter Vakuum zur Trockne eingeengt, in 2 l Wasser aufgenommen, mit 200 ml 2N Natriumhydroxid alkalisch gestellt, mit 200 ml 2N Salzsäure neutralisiert, durch Filtration vom Katalysator befreit und die erhaltene hellgelbe Lösung unter Vakuum zur Trockne eingeengt. Der Rückstand wird in 300 ml Wasser aufgenommen und daraus wird das Reaktionsprodukt durch Zugabe von 34 g Natriumchlorid ausgefällt, abgenutscht, mit 100 ml einer 7,5%igen Natriumchlorid-Lösung gewaschen und bis zum konstanten Gewicht unter Vakuum bei 110°C getrocknet. Man erhält 95,0 g von 4,4'-Dinitrostilben-2,2'-disulfonsäure Dinatriumsalz in Form eines hellgelben kristallinen Pulvers von Schmelzpunkt über 300°C, das einen Aktivgehalt (UV-spektralphotometrisch bestimmt) von 94,0 % aufweist. Die Ausbeute an 4,4'-Dinitrostilben-2,2'-disulfonsäure Dinatriumsalz beträgt 94,1 % der Theorie. Die Ausbeute an 4,4'-Dinitrostilben-2,2'-disulfonsäure Dinatriumsalz bestimmt durch LC-Analyse beträgt 93,6 % der Theorie.

Beispiel 2:

In einem Glasautoklav gemäss Beispiel 1 werden bei atmosphärischem Druck und bei -40°C, 97,0 g 4-Nitrotoluol-2-sulfonsäure Natriumsalz (Aktivgehalt: 98,6 %), 3,1 g Mangan-(II)-sulfat Monohydrat und 360 g flüssiges Ammoniak vorgelegt - dies entspricht einem Verhältnis von 1 Teil p-NTSA-Na-Salz zu 3,8 Teilen Ammoniak. Der Autoklav wird geschlossen und die Innentemperatur von -33,3°C auf +15°C erhöht wobei der Innendruck auf 6 bar steigt.

In die erhaltene klare Lösung wird ein Sauerstoffstrom von 10 l/h, bei einer Rührgeschwindigkeit von 600 bis 700 UpM eingeleitet. Zusätzlich werden 11 g einer 30 %igen methanolischen Natriummethylat-Lösung innerhalb 25 Minuten bei einer Innentemperatur von 15°C zugegeben. Das erhaltene Reaktionsgemisch wird nun eine Stunde und 35 Minuten bei 15°C unter Einleiten von Sauerstoff (10 l/h) weitergerührt.

Die Aufarbeitung erfolgt wie in Beispiel 1 beschrieben, mit der Ausnahme, dass das Reaktionsgemisch mit 4,5 g Ammoniumchlorid versetzt wird. Man erhält 96,2 g von 4,4'-Dinitrostilben-2,2'-disulfonsäure Dinatriumsalz in Form eines hellgelben kristallinen Pulvers vom Schmelzpunkt über 300°C, das einen Aktivgehalt (UV-spektralphotometrisch bestimmt) von 91,7 % aufweist. Die Ausbeute an 4,4'-Dinitrostilben-2,2'-disulfonsäure Dinatriumsalz beträgt 93,0 % der Theorie.

Aehnliche Ergebnisse werden erhalten, wenn man die Reaktion in einem Verhältnis von 1 Teil p-NTSA-Na-Salz zu 2,5 Teilen Ammoniak durchführt.

Beispiel 3:

In einem 1 l BUECHI-Glasautoklav ausgerüstet mit Kühl/Heiz-Mantel, Manometer, Begasungsrührer, Thermometer, Tropftrichter, Begasungsrohr über Niveau, zwei Einwegventile und zwei Rotameter für Ein- und Ausleiten von Sauerstoff und Berstscheibe (10 bar) werden bei atmosphärischem Druck und bei -40°C, 97,0 g 4-Nitrotoluol-2-sulfonsäure Natriumsalz (Aktivgehalt: 98,6 %), 4,6 g Mangan-(II)-nitrat Tetrahydrat und 375 g flüssiges Ammoniak vorgelegt - dies entspricht einem Verhältnis von 1 Teil p-NTSA-Na-Salz zu 3,9 Teilen Ammoniak. Der Autoklav wird geschlossen und die Innentemperatur von -

33,3°C auf +15°C erhöht wobei der Innendruck auf 6 bar steigt.

Ueber Niveau der erhaltenen klaren Lösung wird ein Sauerstoffstrom von 13 l/h, bei einer Rührgeschwindigkeit von 600 bis 700 UpM eingeleitet. Zusätzlich wird eine Lösung von 4,8 g Natriumhydroxid in 24 g Methanol innerhalb 30 Minuten bei einer Innentemperatur von 15°C zugegeben. Das erhaltene Reaktionsgemisch wird nun eine Stunde bei 15°C unter Einleiten von Sauerstoff (13 l/h) weitergerührt.

Die Aufarbeitung erfolgt wie in Beispiel 1 beschrieben. Man erhält 97,2 g von 4,4'-Dinitrostilben-2,2'-disulfonsäure Dinatriumsalz in Form eines hellgelben kristallinen Pulvers vom Schmelzpunkt über 300°C, das einen Aktivgehalt (UV-spektralphotometrisch bestimmt) von 89,1 % aufweist. Die Ausbeute an 4,4'-Dinitrostilben-2,2'-disulfonsäure Dinatriumsalz beträgt 91,3 % der Theorie.

Beispiel 4:

In einem Glasautoklav gemäss Beispiel 3 werden bei atmosphärischem Druck und bei -40°C, 97,0 g 4-Nitrotoluol-2-sulfonsäure Natriumsalz (Aktivgehalt: 98,6 %), 4,5 g Mangan (II)-acetat Tetrahydrat und 360 g flüssiges Ammoniak vorgelegt. Der Autoklav wird geschlossen und die Innentemperatur von -33,3°C auf +15°C erhöht wobei der Innendruck auf 6 bar steigt.

Ueber Niveau der erhaltenen klaren Lösung wird ein Sauerstoffstrom von 13 l/h, bei einer Rührgeschwindigkeit von 600 bis 700 UpM eingeleitet. Zusätzlich werden 19,2 g einer 50 %igen wässrigen Natriumhydroxid-Lösung innerhalb 15 Minuten bei einer Innentemperatur von 15°C zugegeben. Das erhaltene Reaktionsgemisch enthaltend ein Ammoniak/Wasser-Gemisch von 97,5 % Ammoniak zu 2,5 % Wasser (dies entspricht einem Verhältnis von 1 Teil p-NTSA-Na-Salz zu 3,9 Teilen Lösungsmittelgemisch) wird nun eine Stunde und 15 Minuten bei 15°C unter Einleiten von Sauerstoff (13 l/h) weitergerührt.

Die Aufarbeitung erfolgt wie in Beispiel 1 beschrieben. Man erhält 95,2 g von 4,4'-Dinitrostilben-2,2'-disulfonsäure Dinatriumsalz in Form eines hellgelben kristallinen Pulvers vom Schmelzpunkt über 300°C, das einen Aktivgehalt (UV-spektralphotometrisch bestimmt) von 93,5 % aufweist. Die Ausbeute an 4,4'-Dinitrostilben-2,2'-disulfonsäure Dinatriumsalz beträgt 93,8 % der Theorie. Die Ausbeute an 4,4'-Dinitrostilben-2,2'-disulfonsäure Dinatriumsalz bestimmt durch LC-Analyse beträgt 92,8 % der Theorie.

Beispiel 5:

In einem Glasautoklav gemäss Beispiel 1 werden bei atmosphärischem Druck und bei -40°C, 97,0 g 4-Nitrotoluol-2-sulfonsäure Natriumsalz (Aktivgehalt: 98,6 %), 4,5 g Mangan-(II)-acetat Tetrahydrat und 360 g flüssiges Ammoniak vorgelegt. Der Autoklav wird geschlossen und die Innentemperatur von -33,3°C auf +15°C erhöht wobei der Innendruck auf 6 bar steigt.

In die erhaltene klare Lösung wird ein Sauerstoffstrom von 13 l/h, bei einer Rührgeschwindigkeit von 600 bis 700 UpM eingeleitet. Zusätzlich werden 16,0 g einer wässrigen 30 %igen Natriumhydroxid-Lösung innerhalb von 15 Minuten bei einer Innentemperatur von 15 bis 17°C zugegeben. Das erhaltene Reaktionsgemisch enthaltend ein Ammoniak/Wasser-Gemisch von 97 % Ammoniak und 3 % Wasser (dies entspricht einem Verhältnis von 1 Teil p-NTSA-Na-Salz zu 3,9 Teilen Lösungsmittelgemisch) wird nun 45 Minuten bei 15°C unter Einleiten von Sauerstoff (13 l/h) weitergerührt.

Die Aufarbeitung erfolgt wie in Beispiel 1. Man erhält 97,0 g 4,4'-Dinitrostilben-2,2'-disulfonsäure Dinatriumsalz in Form eines hellgelben kristallinen Pulvers vom Schmelzpunkt über 300°C, das einen Aktivgehalt (UV-spektralphotometrisch bestimmt) von 92,2 % aufweist. Die Ausbeute an 4,4'-Dinitrostilben-2,2'-disulfonsäure Dinatriumsalz beträgt 94,3 % der Theorie. Die Ausbeute an 4,4'-Dinitrostilben-2,2'-disulfonsäure Dinatriumsalz bestimmt durch LC-Analyse beträgt 95,0 % der Theorie.

Beispiel 6:

In einem Glasautoklav gemäss Beispiel 3 werden bei atmosphärischem Druck und bei -40°C, 97,0 g 4-Nitrotoluol-2-sulfonsäure Natriumsalz (Aktivgehalt: 98,6 %), 3,1 g Mangan-(II)-sulfat Monohydrat und 360 g flüssiges Ammoniak vorgelegt. Der Autoklav wird geschlossen und die Innentemperatur von -33,3°C auf +15°C erhöht wobei der Innendruck auf 6 bar steigt.

Ueber Niveau der erhaltenen klaren Lösung wird ein Sauerstoffstrom von 13 l/h, bei einer Rührgeschwindigkeit von 600 bis 700 UpM eingeleitet. Zusätzlich werden 32 g einer 15 %igen wässrigen Natriumhydroxid-Lösung innerhalb 15 Minuten bei einer Innentemperatur von 15°C bis 17°C zugegeben. Das erhaltene Reaktionsgemisch enthaltend ein Ammoniak/Wasser-Gemisch von 93 % Ammoniak und 7 % Wasser (dies entspricht einem Verhältnis von 1 Teil p-NTSA-Na-Salz zu 4,1 Teilen Lösungsmittelgemisch) wird nun 45 Minuten bei 15°C unter Einleiten von Sauerstoff (13 l/h) weitergerührt.

Die Aufarbeitung erfolgt wie in Beispiel 1 beschrieben. Man erhält 94,4 g von 4,4'-Dinitrostilben-2,2'-disulfonsäure Dinatriumsalz in Form eines hellgelben kristallinen Pulvers vom Schmelzpunkt über 300°C, das einen Aktivgehalt (UV-spektralphotometrisch bestimmt) von 96,1 % aufweist. Die Ausbeute an 4,4'-Dinitrostilben-2,2'-disulfonsäure Dinatriumsalz beträgt 95,6 % der Theorie. Die Ausbeute an 4,4'-Dinitrostilben-2,2'-disulfonsäure Dinatriumsalz des Reaktionsgemisches vor der Aufarbeitung, bestimmt durch LC-Analyse beträgt 96,4 % der Theorie.

Beispiel 7:

In einem Glasautoklav gemäss Beispiel 3 werden bei atmosphärischem Druck und bei -40°C, 97,0 g 4-Nitrotoluol-2-sulfonsäure Natriumsalz (Aktivgehalt: 98,6 %), 4,6 g Mangan-(II)-nitrat Tetrahydrat und 360 g flüssiges Ammoniak vorgelegt. Der Autoklav wird geschlossen und die Innentemperatur von -33,3°C auf +15°C erhöht wobei der Innendruck auf 6 bar steigt.

Ueber Niveau der erhaltenen klaren Lösung wird ein Sauerstoffstrom von 13 l/h, bei einer Rührgeschwindigkeit von 600 bis 700 UpM eingeleitet. Zusätzlich werden 64 g einer 7,5 %igen wässrigen Natriumhydroxid-Lösung innerhalb 15 Minuten bei einer Innentemperatur von 15°C bis 20°C zugegeben. Das erhaltene Reaktionsgemisch enthaltend ein Ammoniak/Wasser-Gemisch von 86 % Ammoniak und 14 % Wasser (dies entspricht einem Verhältnis von 1 Teil p-NTSA-Na-Salz zu 4,4 Teilen Lösungsmittelgemisch) wird nun 45 Minuten bei 15°C unter Einleiten von Sauerstoff (13 l/h) weitergerührt.

Die Aufarbeitung erfolgt wie in Beispiel 1 beschrieben. Man erhält 93,4 g von 4,4'-Dinitrostilben-2,2'-disulfonsäure Dinatriumsalz in Form eines hellgelben kristallinen Pulvers vom Schmelzpunkt über 300°C, das einen Aktivgehalt (UV-spektralphotometrisch bestimmt) von 96,9 % aufweist. Die Ausbeute an 4,4'-Dinitrostilben-2,2'-disulfonsäure Dinatriumsalz beträgt 95,4 % der Theorie. Die Ausbeute an 4,4'-Dinitrostilben-2,2'-disulfonsäure Dinatriumsalz bestimmt durch LC-Analyse beträgt 94,7 % der Theorie.

Beispiel 8:

In einem Glasautoklav gemäss Beispiel 3 werden bei atmosphärischem Druck 77,6 g 4-Nitrotoluol-2-sulfonsäure Natriumsalz (Aktivgehalt: 98,6 %), 1,6 g Mangan-(II)-acetat Tetrahydrat und 66 g einer 25 %igen wässrigen Ammoniak-Lösung vorgelegt. Der Autoklav wird geschlossen und bei einer Innentemperatur von 15°C werden nun 242 g flüssiges Ammoniak zugegeben, wobei der Innendruck auf 4,4 bar steigt.

Ueber Niveau der erhaltenen klaren Lösung wird ein Sauerstoffstrom von 10 l/h, bei einer Rührgeschwindigkeit von 600 bis 700 UpM eingeleitet. Zusätzlich werden 42,7 g einer 30 %igen wässrigen Natriumhydroxid-Lösung innerhalb 20 Minuten bei einer Innentemperatur von 15°C bis 17°C zugegeben. Das erhaltene Reaktionsgemisch enthaltend ein Ammoniak/Wasser- Gemisch von 76,5 % Ammoniak und 23,5 % Wasser (dies entspricht einem Verhältnis von 1 Teil p-NTSA-Na-Salz zu 4,4 Teilen Lösungsmittelgemisch) wird nun zwei Stunden bei 15°C unter Einleiten von Sauerstoff (10 l/h) weitergerührt.

Die Aufarbeitung erfolgt wie in Beispiel 1 beschrieben mit der Ausnahme, dass das Reaktionsgemisch mit 17 g Ammoniumchlorid versetzt wird und mit 100 ml Wasser anstelle von Methanol verdünnt wird. Man erhält 75,4 g von 4,4'-Dinitrostilben-2,2'-disulfonsäure Dinatriumsalz in Form eines hellgelben kristallinen Pulvers vom Schmelzpunkt über 300°C, das einen Aktivgehalt (UV-spektralphotometrisch bestimmt) von 96,7 % aufweist. Die Ausbeute an 4,4'-Dinitrostilben-2,2'-disulfonsäure Dinatriumsalz beträgt 96,0 % der Theorie. Die Ausbeute an 4,4'-Dinitrostilben-2,2'-disulfonsäure Dinatriumsalz des Reaktionsgemisches vor der Aufarbeitung, bestimmt durch LC-Analyse beträgt 96,5 % der Theorie.

Beispiel 9:

In einem Autoklav gemäss Beispiel 1 werden bei atmosphärischem Druck und bei -40°C, 97,0 g 4-Nitrotoluol-2-sulfonsäure Natriumsalz (Aktivgehalt: 98,6 %), 94,4 g Wasser, 4,5 g Mangan-(II)-acetat Tetrahydrat und 266 g flüssiges Ammoniak vorgelegt. Der Autoklav wird geschlossen und die Innentemperatur von -33,3°C auf +13°C erhöht wobei der Innendruck auf 3,3 bar steigt.

In die erhaltene klare Lösung wird ein Sauerstoffstrom von 10 l/h, bei einer Rührgeschwindigkeit von 600 bis 700 UpM eingeleitet. Zusätzlich werden 16,0 g einer wässrigen 30 %igen Natriumhydroxid-Lösung innerhalb von 15 Minuten bei einer Innentemperatur von 13 bis 15°C zugegeben. Das erhaltene Reaktionsgemisch enthaltend ein Ammoniak/Wasser-Gemisch von 71,5 % Ammoniak und 28,5 % Wasser (dies entspricht einem Verhältnis von 1 Teil p-NTSA-Na-Salz zu 3,9 Teilen Lösungsmittelgemisch) wird nun eine Stunde bei 15°C unter Einleiten von Sauerstoff (10 l/h) weitergerührt.

Die Aufarbeitung wird wie in Beispiel 1 beschrieben durchgeführt, wobei anstelle von 400 ml Methanol, 300 ml Wasser verwendet werden. Man erhält 96,3 g von 4,4'-Dinitrostilben-2,2'-disulfonsäure Dinatriumsalz in Form eines hellgelben kristallinen Pulvers vom Schmelzpunkt über 300°C, das einen Aktivgehalt (UV-spektralphotometrisch bestimmt) von 91,6 % auf weist. Die Ausbeute an 4,4'-Dinitrostilben-2,2'-disulfonsäure Dinatriumsalz beträgt 93,0 % der Theorie. Die Ausbeute an 4,4'-Dinitrostilben-2,2'-disulfonsäure Dinatriumsalz bestimmt durch LC-Analyse beträgt 92,8 % der Theorie.

Werden anstelle von 97,0 g 4-Nitrotoluol-2-sulfonsäure Natriumsalz, 115,1 g dieser Verbindung als feuchter Presskuchen (Aktivgehalt: 83,1 %; Wassergehalt: 15,7 %) und entsprechend 76,3 g Wasser eingesetzt, erhält man die gleichen Ergebnisse.

Beispiel 10:

In einem Glasautoklav gemäss Beispiel 3 werden bei atmosphärischem Druck 97,0 g 4-Nitrotoluol-2-sulfonsäure Natriumsalz (Aktivgehalt: 98,6 %), 2 g Mangan-(II)-acetat Tetrahydrat und 195 g einer 25 %igen wässrigen Ammoniak-Lösung vorgelegt. Der Autoklav wird geschlossen und bei einer Innentemperatur von 15°C werden nun 210 g flüssiges Ammoniak zugegeben, wobei der Innendruck auf 2,6 bar steigt.

Ueber Niveau der erhaltenen klaren Lösung wird ein Sauerstoffstrom von 10 l/h, bei einer Rührgeschwindigkeit von 600 bis 700 UpM eingeleitet. Zusätzlich werden 26,7 g einer 30 %igen wässrigen Natriumhydroxid-Lösung innerhalb 20 Minuten bei einer Innentemperatur von 10°C bis 15°C zugegeben. Das erhaltene Reaktionsgemisch enthaltend ein Ammoniak/Wasser-Gemisch von 61 % Ammoniak und 39 % Wasser (dies entspricht einem Verhältnis von 1 Teil p-NTSA-Na-Salz zu 4,4 Teilen Lösungsmittelgemisch) wird nun zwei Stunden bei 15°C unter Einleiten von Sauerstoff (10 l/h) weitergerührt.

Die Aufarbeitung erfolgt wie in Beispiel 1 beschrieben mit der Ausnahme, dass das Reaktionsgemisch mit 11 g Ammoniumchlorid versetzt wird und mit 100 ml Wasser anstelle von Methanol verdünnt wird. Man erhält 95,4 g von 4,4'-Dinitrostilben-2,2'-disulfonsäure Dinatriumsalz in Form eines hellgelben kristallinen Pulvers vom Schmelzpunkt über 300°C, das einen Aktivgehalt (UV-spektralphotometrisch bestimmt) von 92,4 % aufweist. Die Ausbeute an 4,4'-Dinitrostilben-2,2'-disulfonsäure Dinatriumsalz beträgt 92,9 % der Theorie. Die Ausbeute an 4,4'-Dinitrostilben-2,2'-disulfonsäure Dinatriumsalz des Reaktionsgemisches vor der Aufarbeitung, bestimmt durch LC-Analyse beträgt 95,7 % der Theorie.

Werden anstelle von 97,0 g 4-Nitrotoluol-2-sulfonsäure Natriumsalz, 115,1 g dieser Verbindung als feuchter Presskuchen (Aktivgehalt: 83,1 %; Wassergehalt: 15,7 %) und entsprechend 171 g einer 25 %igen wässrigen Ammoniak-Lösung und 216 g flüssiges Ammoniak eingesetzt, erhält man die gleichen Ergebnisse.

Beispiel 11:

In einem Glasautoklav gemäss Beispiel 3 werden bei atmosphärischem Druck 77,6 g 4-Nitrotoluol-2-sulfonsäure Natriumsalz (Aktivgehalt: 98,6 %), 1,6 g Mangan-(II)-acetat Tetrahydrat und 202 g einer 25 %igen wässrigen Ammoniak-Lösung vorgelegt. Der Autoklav wird geschlossen und bei einer Innentemperatur von 20°C werden nun 210 g flüssiges Ammoniak zugegeben, wobei der Innendruck auf 4 bar steigt.

Ueber Niveau der erhaltenen klaren Lösung wird ein Sauerstoffstrom von 10 l/h, bei einer Rührgeschwindigkeit von 600 bis 700 UpM eingeleitet. Zusätzlich werden 21,3 g einer 30 %igen wässrigen Natriumhydroxid-Lösung innerhalb 20 Minuten bei einer Innentemperatur von 18°C bis 20°C zugegeben. Das erhaltene Reaktionsgemisch enthaltend ein Ammoniak/Wasser-Gemisch von 61 % Ammoniak und 39 % Wasser (dies entspricht einem Verhältnis von 1 Teil p-NTSA-Na-Salz zu 5,6 Teilen Lösungsmittelgemisch) wird nun zwei Stunden bei 20°C unter Einleiten von Sauerstoff (10 l/h) weitergerührt.

Die Aufarbeitung erfolgt wie in Beispiel 1 beschrieben mit der Ausnahme, dass das Reaktionsgemisch mit 9 g Ammoniumchlorid versetzt wird und mit 100 ml Wasser anstelle von Methanol verdünnt wird. Man erhält 76,8 g von 4,4'-Dinitrostilben-2,2'-disulfonsäure Dinatriumsalz in Form eines hellgelben kristallinen Pulvers vom Schmelzpunkt über 300°C, das einen Aktivgehalt (UV-spektralphotometrisch bestimmt) von 94,8 % aufweist. Die Ausbeute an 4,4'-Dinitrostilben-2,2'-disulfonsäure Dinatriumsalz beträgt 95,9 % der Theorie. Die Ausbeute an 4,4'-Dinitrostilben-2,2'-disulfonsäure Dinatriumsalz des Reaktionsgemisches vor der Aufarbeitung, bestimmt durch LC-Analyse beträgt 96,5 % der Theorie.

Aehnliche Ergebnisse werden erhalten, wenn man die Reaktion in einem Verhältnis von 1 Teil p-NTSA-Na-Salz zu 8 Teilen Lösungsmittelgemisch durchführt.

Beispiel 12:

In einem Glasautoklav gemäss Beispiel 3 werden bei atmosphärischem Druck 77,6 g 4-Nitrotoluol-2-sulfonsäure Natriumsalz (Aktivgehalt: 98,6 %), 1,6 g Mangan-(II)-acetat Tetrahydrat und 148 g einer 25 %igen wässrigen Ammoniak-Lösung vorgelegt. Der Autoklav wird geschlossen und bei einer Innentemperatur von15°C werden nun 172 g flüssiges Ammoniak zugegeben, wobei der Innendruck auf 3,5 bar steigt.

Ueber Niveau der erhaltenen klaren Lösung wird ein Sauerstoffstrom von 10 l/h, bei einer Rührgeschwindigkeit von 600 bis 700 UpM eingeleitet. Zusätzlich werden 32 g einer 30 %igen wässrigen Natriumhydroxid-Lösung innerhalb 20 Minuten bei einer Innentemperatur von 10°C bis 15°C zugegeben. Das erhaltene Reaktionsgemisch enthaltend ein Ammoniak/Wasser-Gemisch von 61 % Ammoniak und 39 % Wasser (dies entspricht einem Verhältnis von 1 Teil p-NTSA-Na-Salz zu 4,5 Teilen Lösungsmittelgemisch) wird nun zwei Stunden bei 15°C unter Einleiten von Sauerstoff (10 l/h) weitergerührt.

Die Aufarbeitung erfolgt wie in Beispiel 1 beschrieben mit der Ausnahme, dass das Reaktionsgemisch mit 12,8 g Ammoniumchlorid versetzt wird und mit 100 ml Wasser anstelle von Methanol verdünnt wird. Man erhält 78,2 g von 4,4'-Dinitrostilben-2,2'-disulfonsäure Dinatriumsalz in Form eines hellgelben kristallinen Pulvers vom Schmelzpunkt über 300°C, das einen Aktivgehalt (UV-spektralphotometrisch bestimmt) von 93,4 % aufweist. Die Ausbeute an 4,4'-Dinitrostilben-2,2'-disulfonsäure Dinatriumsalz beträgt 96,2% der Theorie. Die Ausbeute an 4,4'-Dinitrostilben-2,2'-disulfonsäure Dinatriumsalz des Reaktionsgemisches vor der Aufarbeitung, bestimmt durch LC-Analyse beträgt 97,3 % der Theorie.

Beispiel 13:

In einem Glasautoklav gemäss Beispiel 3 werden bei atmosphärischem Druck 97,7 g 4-Nitrotoluol-2-sulfonsäure (Aktivgehalt: 73,4 %; Wassergehalt: 18,2 %; Schwefelsäuregehalt: 8,4 %) und 1,6 g Mangan(II)-acetat Tetrahydrat vorgelegt. Der Autoklav wird geschlossen und bei einer Innentemperatur von 20°C bis 24°C werden innerhalb 35 Minuten 257 g flüssiges Ammoniak zugegeben, wobei der Innendruck bis 5,6 bar steigt. Das Reaktionsgemisch wird nun bei einer Innentemperatur von 10°C bis 15°C innerhalb 15 Minuten mit 64,8 g einer 30 %igen wässrigen Natriumhydroxid-Lösung versetzt.

Ueber Niveau der erhaltenen klaren Lösung wird ein Sauerstoffstrom von 10 l/h, bei einer Rührgeschwindigkeit von 600 bis 700 UpM eingeleitet. Zusätzlich werden 21,3 g einer 30 %igen wässrigen Natriumhydroxid-Lösung innerhalb 20 Minuten bei einer Innentemperatur von 15°C bis 20°C zugegeben. Das erhaltene Reaktionsgemisch enthaltend ein Ammoniak/Wasser-Gemisch von 77 % Ammoniak und 23 % Wasser (dies entspricht einem Verhältnis von 1 Teil p-NTSA zu 4,7 Teilen Lösungsmittelgemisch) wird nun zwei Stunden bei 15°C unter Einleiten von Sauerstoff (10 l/h) weitergerührt.

Die Aufarbeitung erfolgt wie in Beispiel 1 beschrieben mit der Ausnahme, dass das Reaktionsgemisch mit 8,6 g Ammoniumchlorid versetzt wird und mit 100 ml Wasser anstelle von Methanol verdünnt wird. Man erhält 76,1 g von 4,4'-Dinitrostilben-2,2'-disulfonsäure Dinatriumsalz in Form eines hellgelben kristallinen Pulvers vom Schmelzpunkt über 300°C, das einen Aktivgehalt (UV-spektralphotometrisch bestimmt) von 93,8 % aufweist. Die Ausbeute an 4,4'-Dinitrostilben-2,2'-disulfonsäure Dinatriumsalz beträgt 94,0 % der Theorie. Die Ausbeute an 4,4'-Dinitrostilben-2,2'-disulfonsäure Dinatriumsalz des Reaktionsgemisches vor der Aufarbeitung, bestimmt durch LC-Analyse beträgt 94,5 % der Theorie.

Beispiel 14:

In einem Glasautoklav gemäss Beispiel 3 werden bei atmosphärischem Druck und bei -10°C, 77,6 g 4-Nitrotoluol-2-sulfonsäure Natriumsalz (Aktivgehalt: 98,6 %), 3,2 g Mangan(II)-acetat Tetrahydrat und 415 g flüssiges Methylamin vorgelegt - dies entspricht einem Verhältnis von 1 Teil p-NTSA-Na-Salz zu 5,5 Teilen Lösungsmittel.

Ueber Niveau der erhaltenen klaren Lösung wird ein Sauerstoffstrom von 8 l/h, bei einer Rührgeschwindigkeit von 1800 UpM eingeleitet. Zusätzlich werden 28,8 g einer 30 %igen methanolischen Natriummethylat-Lösung innerhalb 20 Minuten bei einer Innentemperatur von -10°C bis -5°C zugegeben. Das erhaltene Reaktionsgemisch wird nun eine Stunde und 40 Minuten bei -10°C unter Einleiten von Sauerstoff (8 l/h) weitergerührt.

Die Aufarbeitung erfolgt wie in Beispiel 1 beschrieben mit der Ausnahme, dass das Reaktionsgemisch mit 8,6 Ammoniumchlorid versetzt wird und mit 200 ml Methanol verdünnt wird. Man erhält 61,5 g von 4,4'-Dinitrostilben-2,2'-disulfonsäure Dinatriumsalz in Form eines hellgelben kristallinen Pulvers vom Schmelzpunkt über 300°, das einen Aktivgehalt (UV-spektralphotometrisch bestimmt) von 92,3 % aufweist. Die Ausbeute an 4,4'-Dinitrostilben-2,2'-disulfonsäure Dinatriumsalz beträgt 74,8 % der Theorie.

**Patentansprüche**

1. Verfahren zur Herstellung von 4,4'-Dinitrostilben-2,2'-disulfonsäure und deren Salze der Formel

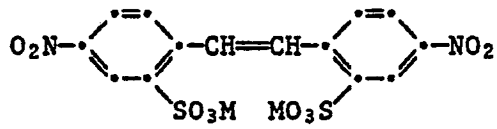

worin M Wasserstoff, ein Alkalimetallkation oder ein Ammoniumkation bedeutet, durch Oxidation von 4-Nitrotoluol-2-sulfonsäure oder deren Salze mit einem Oxidationsmittel, dadurch gekennzeichnet, dass man die Oxydation in flüssigem, wasserfreiem Ammoniak, dessen Alkylderivat gegebenenfalls in Gegenwart von Wasser und/oder deren Mischungen untereinander in Gegenwart von starken Basen durchführt.

2. Verfahren gemäss Anspruch 1, dadurch gekennzeichnet, dass die Oxydation in einem Gemisch von Ammoniak mit Wasser durchgeführt wird.

3. Verfahren gemäss Anspruch 1, dadurch gekennzeichnet, dass die Base in einem protischen Lösungsmittel gelöst eingesetzt wird.

4. Verfahren gemäss Anspruch 3, dadurch gekennzeichnet, dass als protisches Lösungsmittel ein niedermolekularer Alkohol und/oder Wasser verwendet wird.

5. Verfahren gemäss Anspruch 1, dadurch gekennzeichnet, das als starke Base eine Alkali- oder Erdalkaliverbindung verwendet wird.

6. Verfahren gemäss Anspruch 5, dadurch gekennzeichnet, dass als starke Base Hydroxide und Alkoholate von Alkali- oder Erdalkalimetallen oder Gemische aus solchen Verbindungen, sowie stark basische Ionenaustauscher verwendet werden.

7. Verfahren nach Anspruch 1, dadurch gekennzeichnet, dass zusätzlich Katalysatoren verwendet werden.

8. Verfahren gemäss Anspruch 7, dadurch gekennzeichnet, dass als Katalysatoren, Salze, Oxide oder Hydroxide von Schwermetall-Verbindungen und/oder schwermetallorganischen Verbindungen verwendet werden.

9. Verfahren gemäss Anspruch 7, dadurch gekennzeichnet, dass als Katalysatoren Phasentransferkatalysatoren oder Kronenäther verwendet werden.

10. Verfahren gemäss Anspruch 1, dadurch gekennzeichnet, dass die Oxydation bei Temperaturen zwischen -33°C und +50°C durchgeführt wird.

11. Verfahren gemäss Anspruch 10, dadurch gekennzeichnet, dass die Oxydation bei Temperaturen zwischen -15°C und 30°C durchgeführt wird.

12. Verfahren gemäss Anspruch 1, dadurch gekennzeichnet, dass 1 bis 10 Teile Ammoniak, Alkylderivat gegebenenfalls in Gegenwart von Wasser bzw. deren Mischungen pro Teil 4-Nitrotoluol-2-sulfonsäure verwendet wird.

13. Verfahren gemäss Anspruch 12, dadurch gekennzeichnet, dass 3 bis 6 Teile Ammoniak, Alkylderivat gegebenenfalls in Gegenwart von Wasser bzw. deren Mischungen pro Teil 4-Nitrotoluol-2-sulfonsäure verwendet wird.

14. Verfahren gemäss Anspruch 1, dadurch gekennzeichnet, dass als Oxydationsmittel reiner Sauerstoff oder seine Gemische mit inerten Gasen oder Luft verwendet werden.

15. Verfahren gemäss Anspruch 1, dadurch gekennzeichnet, dass 1 Teil 4-Nitrotoluol-2-sulfonsäure bzw. deren Salze in Form des feuchten Presskuchens mit einer katalytischen Menge Mangan-II-Salz in 0,5 bis 3 Teilen wässriger Ammoniaklösung oder Wasser suspendiert und mit 1 bis 5 Teilen flüssigem Ammoniak versetzt wird, so dass der Ammoniakgehalt der gesamten Reaktionsmischung 60 % bis 80 % beträgt und diese Mischung in Gegenwart von Sauerstoff als Oxidationsmittel und Natriumhydroxid als Base bei Temperaturen von 0 bis 25°C und unter Druck umsetzt.

16. Verfahren gemäss Anspruch 1, dadurch gekennzeichnet, dass der Anteil von Ammoniak oder den Alkylderivaten des Ammoniaks im Gemisch mit Wasser 50 % bis 99 % beträgt.

17. Verfahren gemäss Anspruch 16, dadurch gekennzeichnet, dass der Anteil von Ammoniak oder den Alkylderivaten des Ammoniaks im Gemisch mit Wasser 60 % bis 80 % beträgt.

**Revendications**

1. Procédé de préparation de l'acide 4,4'-dinitrostilbène-2,2'-disulfonique et de ses sels de formule

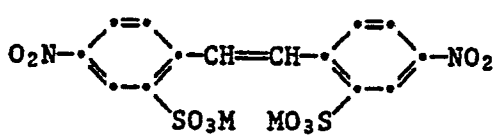

dans laquelle M est un atome d'hydrogène, un cation de métal alcalin ou un cation ammonium, par oxydation de l'acide 4-nitrotoluène-2-sulfonique ou ses sels avec un oxydant, caractérisé en ce que l'on effectue l'oxydation en présence de bases fortes, dans l'ammoniac anhydre liquide, un de ses dérivés alkylés éventuellement en présence d'eau, et/au leurs mélanges.

2. Procédé selon la revendication 1, caractérisé en ce que l'on effectue l'oxydation dans un mélange d'ammoniac et d'eau.

3. Procédé selon la revendication 1, caractérisé en ce que la base est mise en œuvre sous forme dissoute dans un solvant protique.

4. Procédé selon la revendication 3, caractérisé en ce que l'on utilise comme solvant protique un alcool de faible masse moléculaire et/ou de l'eau.

5. Procédé selon la revendication 1, caractérisé en ce que l'on utilise comme base forte un composé alcalin ou alcalino-terreux.

6. Procédé selon la revendication 5, caractérisé en ce que l'on utilise comme base forte des hydroxydes ou alcoolates de métaux alcalins ou alcalino-terreux, ou des mélanges desdits composés, ainsi que des échangeurs d'ions fortement basiques.

7. Procédé selon la revendication 1, caractérisé en ce que l'on utilise en outre des catalyseurs.

8. Procédé selon la revendication 7, caractérisé en ce que l'on utilise comme catalyseurs des sels, des oxydes et des hydroxydes de composés de métaux lourds et/ou de composés organométalliques de métaux lourds.

9. Procédé selon la revendication 7, caractérisé en ce que l'on utilise comme catalyseurs des catalyseurs de transfert de phases ou des éthers de couronnes.

10. Procédé selon la revendication 1, caractérisé en ce que l'oxydation est effectuée à des températures comprises entre −33°C et +50°C.

11. Procédé selon la revendication 10, caractérisé en ce que l'oxydation est effectuée à des températures comprises entre −15°C et +30°C.

12. Procédé selon la revendication 1, caractérisé en ce que l'on utilise 1 à 10 parties d'ammoniac, de dérivé alkylé éventuellement en présence d'eau, ou de leurs mélanges, pour 1 partie d'acide 4-nitrotoluène-2-sulfonique.

13. Procédé selon la revendication 12, caractérisé en ce que l'on utilise 3 à 6 parties d'ammoniac, de dérivé alkylé éventuellement en présence d'eau, ou de leurs mélanges, pour 1 partie d'acide 4-nitrotoluène-2-sulfonique.

14. Procédé selon la revendication 1, caractérisé en ce que l'on utilise comme oxydant de l'oxygène pur ou ses mélanges avec des gaz inertes ou l'air.

15. Procédé selon la revendication 1, caractérisé en ce que l'on met en suspension 1 partie d'acide 4-nitrotoluène-2-sulfonique ou de ses sels sous forme de gâteau de filtration humide avec une quantité catalytique de sel de manganèse (II) dans 0,5 à 3 parties de solution hydroammoniacale ou d'eau, que l'on mélange le tout avec 1 à 5 parties d'ammoniac liquide, de manière à ce que la proportion d'ammoniac dans le mélange réactionnel total atteigne de 60% à 80%, et que l'on fait réagir ce mélange, en présence d'oxygène comme oxydant et d'hydroxyde de sodium comme base, à des températures comprises entre 0 et 25°C et sous pression.

16. Procédé selon la revendication 1, caractérisé en ce que la proportion d'ammoniac ou de dérivés alkylés de l'ammoniac mélangés à l'eau est comprise entre 50% et 99%.

17. Procédé selon la revendication 1, caractérisé en ce que la proportion d'ammoniac ou de dérivés alkylés de l'ammoniac mélangés à l'eau est comprise entre 60% et 80%.

## Claims

1. A process for the preparation of 4, 4'-dinitrostilbene-2, 2'-disulfonic acid or a salt thereof of the formula

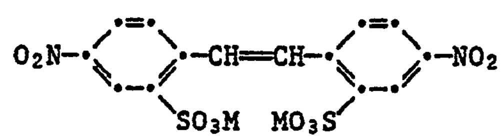

in which M is hydrogen, an alkali metal cation or an ammonium cation, by oxidation of 4-nitrotoluene-2-sulfonic acid or salts thereof with an oxidizing agent, which comprises performing the oxidation in liquid, anhydrous ammonia, in an alkyl derivative thereof in the absence or presence of water and/or mixtures of these solvents in one another and in the presence of strong bases.

2. A process according to claim 1, wherein the oxidation is carried out in a mixture of ammonia with water.

3. A process according to claim 1, wherein the base is used dissolved in a protic solvent.

4. A process according to claim 3, wherein the protic solvent used is a low-molecular-weight alcohol and/or water.

5. A process according to claim 1, wherein the strong base used is an alkali metal or alkaline earth metal compound.

6. A process according to claim 5, wherein the strong base used is a hydroxide or an alcoholate of an alkali metal or an alkaline earth metal or a mixture of these compounds or also a strongly basic ion-exchanger.

7. A process according to claim 1, wherein additionally a catalyst is used.

8. A process according to claim 7, wherein the catalyst used is a salt, oxide or hydroxide of a heavy metal compound and/or an organometallic compound of heavy metal.

9. A process according to claim 7, wherein the catalyst used is a phase transfer catalyst or a crown ether.

10. A process according to claim 1, wherein the oxidation is carried out at temperatures between −33°C and +50°C.

11. A process according to claim 10, wherein the oxidation is carried out at temperatures between −15°C and +30°C.

12. A process according to claim 1, wherein 1 to 10 parts of ammonia, an alkyl derivative thereof in the absence or presence of water or a mixture thereof is used per part of 4-nitrotoluene-2-sulfonic acid.

13. A process according to claim 12, wherein 3 to 6 parts of ammonia, alkyl derivative in the absence or presence of water or a mixture thereof is used per part of 4-nitrotoluene-2-sulfonic acid.

14. A process according to claim 1, wherein the oxidizing agent used is pure oxygen or a mixture thereof with inert gases or is air.

15. A process according to claim 1, wherein 1 part of 4-nitrotoluene-2-sulfonic acid or a salt thereof in the form of a moist presscake is suspended together with a catalytic amount of manganese (II) salt in 0.5 to 3 parts of aqueous ammonia solution or water and are treated with 1 to 5 parts of liquid ammonia, bringing the ammonia content of the entire reaction mixture to 60% to 80% and this mixture is reacted in the presence of oxygen as the oxidizing agent and sodium hydroxide as the base at temperatures from 0 to 25°C and under pressure.

16. A process according to claim 1, wherein the proportion of ammonia or the alkyl derivative of ammonia in a mixture with water is 50% to 99%.

17. A process according to claim 16, wherein the proportion of ammonia or the alkyl derivative of ammonia in a mixture with water is 60% to 80%.